# EUROPEAN PATENT SPECIFICATION

(11) **EP 0 454 220 B1**
(45) Date of publication and mention of the grant of the patent: **18.08.1993**
(21) Application number: 91200895.0
(22) Date of filing: 16.04.1991
(51) Int. Cl.: C07H 15/04, C07H 11/00, A61K 31/70

(54) **Carbohydrate derivatives comprising a trisaccharide unit**
Eine Trisaccharideinheit enthaltende Kohlenhydratderivate
Dérivés d'hydrates de carbone comprenant une unité trisaccharide

(30) Priority: 23.04.1990 EP 90201006
(43) Date of publication of application: 30.10.1991
(73) Proprietor: Akzo Nobel N.V., 6824 BM Arnhem (NL); ELF SANOFI, 75008 Paris (FR)
(72) Inventor: van Boeckel, Constant Adriaan Anton, NL-5346 LX OSS (NL); Petitou, Maurice, F-75013 Paris (FR)
(74) Representative: Hermans, Franciscus G.M.

(56) References cited:
- EP-A- 0 301 618

## Description

The invention concerns a carbohydrate derivative comprising a trisaccharide unit of the general formula I 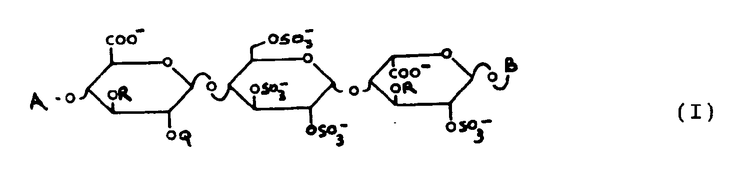
in which R is alkyl, aryl, or aralkyl;
Q is alkyl, aryl, aralkyl, or SO₃⁻;
A and B are independently selected from hydrogen, alkyl, aryl, aralkyl, or a carbohydrate group;
and the charged moieties are compensated by counter-ions.

The invention also deals with a process for the preparation of said carbohydrate derivative, as well as with pharmaceutical compositions containing the same.

The carbohydrate derivatives of this invention possess anti-thrombotic activity, and especially they have potent anti-Xₐ activity without activating thrombin via anti-thrombin-III (AT-III) and they inhibit smooth muscle cell proliferation.

Related carbohydrates are known from European patent application 0,301,618. The compounds described therein are sulfated pentasaccharides DEFGH (letter code according to EP 0,301,618). The trisaccharide unit of the instant compounds corresponds with the EFG part of the prior art compounds, but differs with it, in that free hydroxy groups of the uronic acids E and G are alkylated, arylated, or aralkylated.

It has now been found that the compounds of this invention have a better binding affinity to antithrombin III, which results in a better pharmacokinetic profile, longer half-life times, better specificity, and lower therapeutic doses and thus lesser side-effects. Furthermore, the compounds of this invention have a substantially better heparin cofactor II (HCII) mediated antithrombin activity, and are, therefore, more effective as thrombin generation inhibitors than the prior art compounds. The inclusion of alkyl, aryl, or aralkyl functionalized uronic acids gives further a very important synthetic advantage over the prior art compounds. By functionalizing the hydroxy groups with alkyl, aryl, or aralkyl groups, it is in most cases redundant to prepare temporarily protected carbohydrates, which makes the synthetic pathway considerably shorter and simpler, whereas the replacement of the glucosamine unit F by a glucose unit further simplifies the synthesis of the trisaccharide unit significantly. Moreover, an additional advantage of the synthesis of the compounds of the invention is that the nature of the temporarily protective groups, which are necessary for the protection of the hydroxy groups to be sulfated, is not critical. In this respect it is of interest to note that the biological activities of compounds with Q is alkyl, aryl, or aralkyl and with Q is SO₃⁻ are more or less equal, thus making it less critical to use e.g. benzyl or acyl protection on position 2 of the glucuronic acid residue. Because 2-acyl protection of the glucuronic acid facilitates β coupling of this residue to a glucose unit, compounds of formula (I) having Q is SO₃⁻ are frequently preferred.

The alkyl group in the definition of R, Q, A, and B is a branched or unbranched alkyl group having 1-20 carbon atoms. Alkyl groups for different groups R may be different. Examples are methyl, ethyl, isopropyl, butyl, *sec*-butyl, pentyl, neopentyl, hexyl, octyl, decyl, undecyl, dodecyl, tridecyl, tetradecyl, pentadecyl, hexadecyl, octadecyl, and eicosyl. Preferred are the alkyl groups having 1-6 carbon atoms. More preferred are the alkyl groups having 1-4 carbon atoms, and most preferred is a methyl group. The preferred group Q is methyl or SO₃⁻.

The term aryl in the definition of R, Q, A, and B means an aromatic group, preferably phenyl, which may be substituted by OH, alkyl having 1-4 carbon atoms, alkoxy having 1-4 carbon atoms, halogen (preferably fluorine, chlorine, or bromine), or NR'R'', wherein R' and R'' are independently selected from hydrogen, alkyl having 1-4 carbon atoms, CF₃, benzyloxycarbonyl, carboxyl, and SO₃⁻.

The term aralkyl means an aralkyl group in which the alkyl moiety is an alkyl group having 1-4 carbon atoms and the aryl moiety is an aryl group as previously defined.

The alkyl group having 1-4 carbon atoms is methyl, ethyl, propyl, isopropyl, butyl, *sec*-butyl, or *tert-*butyl, and the alkyl moiety of the alkoxy group having 1-4 carbon atoms has the same meaning.

The term carbohydrate group in the definition of A and B means a mono- or disaccharide, which optionally may be sulfated, or may be protected by alkyl, aryl, or aralkyl groups as previously defined. Suitable carbohydrate groups are, for instance, free, sulfated, alkylated, arylated, or aralkylated derivatives of glucose, mannose, galactose, glucosamine, galactosamine, glucuronic acid, iduronic acid, xylose, arabinose or disaccharides thereof. Preferably, A represents a glucose derivative having the structure II: 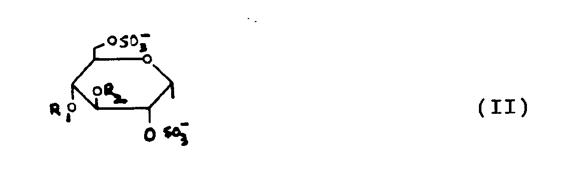
and B represents a glucose derivative having the structure III: 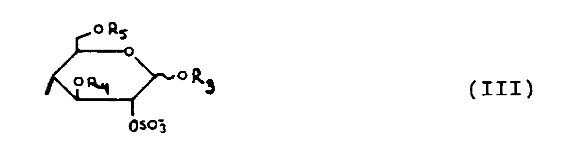
in which R₁, R₂, R₃, R₄ and R₅ are independently selected from hydrogen, alkyl, aryl, aralkyl, or SO₃⁻, whereas alkyl, aryl, and aralkyl has the previously given meanings, or their derivatives in which the charged moieties are compensated by counter-ions. Very suitable derivatives II and III are those in which R₁, R₂, and R₃ are methyl, R₄ and R₅ are SO₃⁻, and OR₃ adopts preferably the α-configuration. Another suitable group A has structure II in which R₁ is methyl, and R₂ is SO₃⁻, and another suitable group B has structure III in which R₄ and R₅ are SO₃⁻, and R₃ is an alkyl group with 6-20 carbon atoms.

It is generally believed that multipoint key polar interactions are of essential importance throughout molecular biology for ensuring high selectivity in noncovalent molecular associations, and that substitution of only one of the key hydroxy groups of an oligosaccharide by a hydrophobic group (and invariably a number of the hydroxy groups prove outstandingly essential to complex formation) can result in complete loss of the affinity by the protein. Remarkably, however, the preferred compounds of this invention having O-alkyl and O-sulfate groups without having free hydroxy groups, still show the full-blown activity.

The counter-ions which compensate the charged moieties are pharmaceutically acceptable counter-ions, like hydrogen, or more preferably alkali or earth-alkali metal ions, like sodium or calcium.

The carbohydrates according to this invention may be prepared according to well known methods described and used for the synthesis of polysaccharides. In this respect, particular reference is made to the previously mentioned European patent 0,301,618, in which methods for the synthesis of polysaccharides are disclosed.

A stepwise condensation of the monosaccharides and the groups A and B is possible. In general, however, building blocks consisting of D-glucose, L-idose, D-glucuronic acid or L-iduronic acid, suitably functionalized with the required alkyl, aryl, or aralkyl groups or by temporarily protective groups, are condensed together in the desired order. In this way the (protected) trisaccharide unit can be prepared, which can be coupled with A and B, or protected derivatives thereof. It is also possible to condense a part of the trisaccharide unit with B or a protected derivative thereof, after which the compound obtained is coupled with the remaining part of the trisaccharide, which may already be provided with group A or a protected derivative thereof. If A is not yet attached to the remaining part of the trisaccharide, the condensed saccharide obtained can be coupled to A (or a protected derivative thereof) in a manner known *per se*. In a similar manner, a part of the (protected) trisaccharide unit containing A can be coupled to the other part, which may be provided with B (or a protected derivative thereof), after which, if B is not present, the coupling product is condensed with B or a protected derivative thereof. Suitable protective groups are well known in carbohydrate chemistry. Preferred protective groups include benzyl and acetyl for hydroxy groups, and methyl and benzyl for the carboxylate group of uronic acids. Other protective groups like levulinoyl, chloroacetyl, trityl, benzoyl, etc, may be used with equal success. Coupling of the saccharides is performed in a manner known in the art, e.g. deprotection of the 1-position of the glycosyl-donor, activation of this position (e.g. by making a bromide, pentenyl, fluoride or trichloroacetamide derivative) and coupling the activated glycosyl-donor with an optionally protected glycosyl-acceptor.

This process of stepwise or building block synthesis affords a protected carbohydrate derivative comprising a trisaccharide unit of the general formula: 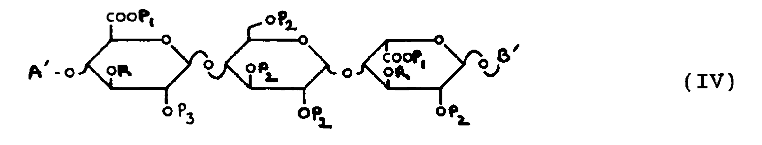
in which R is alkyl, aryl, or aralkyl as previously defined, P₁ is an acid protective group (preferably methyl or benzyl), P₂ is a hydroxy protective group (preferably benzyl or acetyl), P₃ is a hydroxy protective group or the required alkyl, aryl, or aralkyl group, and A' and B' are A and B respectively, or protected derivatives thereof. Carbohydrate derivative IV can be deprotected and sulfated in a manner as described in the previously mentioned EP 0,301,618, in order to obtain the carbohydrate derivative according to formula I. Suitable deprotection methods are, for example, basic hydrolysis for acetyl- and methyl-esters, and hydrogenolysis for benzyl ethers. Sulfation can successfully be performed with complexes of sulfur trioxide with bases like trimethylamine, triethylamine or pyridine in a suitable solvent.

For the treatment of venous thrombosis or for the inhibition of smooth muscle cell proliferation the compounds of the invention may be administered enterally or parenterally, and for humans preferably in a daily dosage of 0,001-10 mg per kg body weight. Mixed with pharmaceutically suitable auxiliaries, the compounds may be compressed into solid dosage units, such as pills, tablets, or be processed into capsules or suppositories. By means of pharmaceutically suitable liquids the compounds can also be applied as an injection preparation in the form of a solution, suspension, emulsion, or as a spray, e.g. a nasal spray.

The invention is further illustrated by the following examples.

### Example 1

### methyl 0-3,4-di-O-methyl-2,6-di-O-sulfo-α-D-glucopyranosyl-(1→4)-O-2,3-di-O-methyl-β-D-glucopyranuronosyl-(1→4)-O-2,3,6-tri-O-sulfo-α-D-glucopyranosyl-(1→4)- O-3-O-methyl-2-O-sulfo-α-L-idopyranuronosyl-(1→4)-O-2,3,6-tri-O-sulfo-α-D-glucopyranoside undecakis sodium salt

a. methyl 6-O-acetyl-3,4-di-O-methyl-2-O-phenylmethyl-α-D-glucopyranosyl-(1→4)-O-(methyl 2,3-di-O-methyl-β-D-glucopyranosyluronate)-(1→4)-O-3,6-di-O-acetyl-2-O-phenylmethyl-α-D-glucopyranosyl-(1→4)-O-(methyl 2-O-acetyl-3-O-methyl-α-L- idopyranosyluronate)-(1→4)-O-2,3,6-tri-O-acetyl-α-D-glucopyranoside (0,061 mmol; prepared according to the methods described in EP 84,999) was dissolved in 4,2 ml of chloroform and added to a mixture of 3 ml of chloroform, 18 ml of methanol and 3,7 ml of 4N sodium hydroxide. The mixture was stirred for 20 h at room temperature and after neutralizing with a diluted aqueous hydrochloric acid solution, evaporated to dryness. The residue was treated with methanol and the insoluble salts were filtered off. The filtrate was evaporated to dryness to obtain methyl 0-3,4-di-O-methyl-2-O-phenylmethyl-α-D-glucopyranosyl-(1→4)-O-2,3-di-O-methyl-β-D-glucopyranuronosyl-(1→4)-O-2-O- phenylmethyl-α-D-glucopyranosyl-(1-4)-O-3-O-methyl-α-L-idopyranuronosyl-(1→4)-O-α-D-glucopyranoside disodium salt.
b. Crude methyl 0-3,4-di-O-methyl-2-O-phenylmethyl-α-D-glucopyranosyl-(1→4)-O-2,3-di-O-methyl-β-D-glucopyranuronosyl-(1→4)-O-2-O-phenylmethyl-α-D- glucopyranosyl-(1→4)-O-3-O-methyl-α-L-idopyranuronosyl-(1→4)-O-α-D-glucopyranoside disodium salt was dissolved in a mixture of 3 ml of water and 1 ml of methanol and 50 mg of 10% Pd/C were added. The mixture was stirred under an atmosphere of hydrogen for 24 h at room temperature. After filtration the filtrate was evaporated to dryness, dissolved in water and desalted on Sephadex G-25. The combined fractions containing the pentasaccharide were evaporated to dryness to give methyl 3,4-di-O-methyl-α-D-glucopyranosyl-(1→4)-O-2,3-di-O-methyl-β-D-glucopyranuronosyl-(1→4)-O-α-D-glucopyranosyl-(1→4)- O-3-O-methyl-α-L-idopyranuronosyl-(1→4)- O-α-D-glucopyranoside disodium salt
c. methyl 0-3,4-di-O-methyl-α-D-glucopyranosyl-(1→4)-O-2,3-di-O-methyl-β-D-glucopyranuronosyl-(1→4)-O-α-D-glucopyranosyl-(1→4)-O-3-O-methyl-α-L- idopyranuronosyl-(1→4)-O-α-D-glucopyranoside di sodium salt was dissolved in a mixture of 2,5 ml of dry dimethylformamide and 1,4 mmol triethylamine sulfurtrioxide complex. The mixture was stirred for 20 h at 50 °C, after which the mixture was cooled to room temperature and a mixture of 500 mg of sodium hydrogen carbonate in 7 ml of water was added. The mixture was stirred for 30 min and then evaporated to dryness. The residue was dissolved in water, desalted on Sephadex G-25, after which the combined fractions were freeze-dried to give amorphous methyl O-3,4-di-O-methyl-2,6-di-O-sulfo-α-D-glucopyranosyl-(1→4)-O-2,3-di-O-methyl-β-D-glucopyranuronosyl-(1→4)-O-2,3,6-tri-O-sulfo- α-D-glucopyranosyl-(1→4)-O-3-O-methyl-2-O-sulfo-α-L-idopyranuronosyl-(1→4)-O-2,3,6-tri-O-sulfo-α-D-glucopyranoside undecakis sodium salt. [α]_{D}²⁰ = +51,3° (c 0,4; water). Anomeric protons chemical shifts: 4.68; 5.14; 5.25; 5.28; and 5.40 ppm.

### Example 2

In a similar manner as described in Example 1 can be prepared:
methyl O-2,3,4,6-tetra-O-sulfo-α-D-glucopyranosyl-(1→4)-O-2,3-di-O-methyl-β-D-glucopyranuronosyl-(1→4)-O-2,3,6-tri-O-sulfo-α-D-glucopyranosyl-(1→4)-O-3-O- methyl-2-O-sulfo-α-L-idopyranuronosyl-(1→4)-O-2,3,6-tri-O-sulfo-α-D-glucopyranoside tridecakis sodium salt. [α]_{D}²⁰ = +44.5° (c 1; water). Anomeric protons chemical shifts: 4.52; 4.79; 4.97; 5.36; and 5.48 ppm.
methyl 0-2,3,4,6-tetra-O-sulfo-α-D-glucopyranosyl-(1→4)-O-3-O-methyl-2-O-sulfo-β-D-glucopyranuronosyl-(1→4)-O-2,3,6-tri-O-sulfo-α-D-glucopyranosyl- (1→4)-O-3-O-methyl-2-O-sulfo-α-L-idopyranuronosyl-(1→4)-O-2,3,6-tri-O-sulfo-α-D-glucopyranoside tetradecakis sodium salt. [α]_{D}²⁰ = +45.1° (c 1; water). Anomeric protons chemical shifts: 4.76; 5.14; 5.15; 5.48; and 5.64 ppm.
methyl 0-3,4-di-O-tetradecyl-2,6-di-O-sulfo-α-D-glucopyranosyl-(1→4)-O-2,3-di-O-methyl-β-D-glucopyranuronosyl-(1→4)-O-2,3,6-tri- O-sulfo-α-D-glucopyranosyl-(1→4)-O-3-O-methyl-2-O-sulfo-α-L-idopyranuronosyl-(1→4)-O-2,3,6-tri-O-sulfo-α-D-glucopyranoside undecakis sodium salt. [α]_{D}²⁰ = +39.0° (c 1; water). Anomeric protons chemical shifts: 4.66; 5.09; 5.24; 5.47; and 5.57 ppm.
methyl 0-3,4-di-O-n-hexyl-2,6-di-O-sulfo-α-D-glucopyranosyl-(1→4)-O-2,3-di-O-methyl-β-D-glucopyranuronosyl-(1→4)-O-2,3,6-tri-O-sulfo-α- D-glucopyranosyl-(1→4)-O-3-O-methyl-2-O-sulfo-α-L-idopyranuronosyl-(1→4)-O-2,3,6-tri-O-sulfo-α-D-glucopyranoside undecakis sodium salt. [α]_{D}²⁰ = +40.8° (c 1; water). Anomeric protons chemical shifts: 4.68; 5.14; 5.16; 5.52; and 5.56 ppm.
methyl 0-3,4-di-O-methyl-2,6-di-O-sulfo-α-D-glucopyranosyl-(1→4)-O-3-O-methyl-2-O-sulfo-β-D-glycopyranuronosyl-(1→4)-O-2,3,6-tri-O- sulfo-α-D-glucopyranosyl-(1→4)-O-3-O-methyl-2-O-sulfo-α-L-idopyranuronosyl-(1→4)-O-2,3,6-tri-O-sulfo-α-D-glucopyranoside dodecakis sodium salt. [α]_{D}²⁰ = +38.5° (c 1; water). Anomeric protons chemical shifts: 5.13; 5.14; 5.31; and 5.56 ppm.
methyl 0-[(4-phenylmethoxycarbonylamino)phenyl]-2,3,6-tri-O-sulfo-α-D-glucopyranosyl-(1→4)-O-2,3-di-O-methyl-β-D-glucopyranuronosyl- (1→4)-O-2,3,6-tri-O-sulfo-α-D-glucopyranosyl-(1→4)-O-3-O-methyl-2-O-sulfo-α-L-ido-pyranuronosyl-(1→4)-O-2,3,6-tri-O-sulfo-α-D-glucopyranoside dodecakis sodium salt. [α]_{D}²⁰ = +56.0° (c 1; water). Anomeric protons chemical shifts: 4.69; 5.16; 5.54; and 5.67 ppm.
methyl 0-(4-aminophenyl)-2,3,6-tri-O-sulfo-α-D-glucopyranosyl-(1→4)-O-2,3-di-O-methyl-β-D-glucopyranuronosyl-(1→4)-O-2,3,6-tri-O-sulfo-α-D- glucopyranosyl-(1→4)-O-3-O-methyl-2-O-sulfo-α-L-idopyranuronosyl-(1→4)-O-2,3,6-tri-O-sulfo-α-D-glucopyranoside dodecakis sodium salt. [α]_{D}²⁰ = +56.0° (c 1; water). Anomeric protons chemical shifts: 4.69; 5.14; 5.16; 5.52; and 5.65 ppm.
methyl 0-(4-sulfonaminophenyl)-2,3,6-tri-O-sulfo-α-D-glucopyranosyl-(1→4)-O-2,3-di-O-methyl-β-D-glucopyranuronosyl-(1→4)-O-2,3,6-tri-O-sulfo-α-D-glucopyranosyl-(1→4)-O-3-O- methyl-2-O-sulfo-α-L-idopyranuronosyl-(1→4)-O-2,3,6-tri-O-sulfo-α-D-glucopyranoside tridecakis sodium salt. [α]_{D}²⁰ = +57.0° (c 1; water). Anomeric protons chemical shifts: 4.69; 5.13; 5.13; 5.53; and 5.67 ppm.

### Example 3

### methyl 0-3,4-di-0-methyl-2,6-di-0-sulfo-α-D-glucopyranosyl-(1→4)-0-2,3-di-0-methyl-β-D-glucopyranuronosyl-(1→4)-0-2,3,6-tri-0-sulfo-α-D-glucopyranosyl-(1→4)- 0-3-0-octyl-2-0-sulfo-α-L-idopyranuronosyl-(1→4)-0-2,3,6-tri-0-sulfo-α-D-glucopyranoside undecakis sodium salt.

a. methyl 0-6-0-acetyl-3,4-di-0-methyl-2-0-phenylmethyl-α-D-glucopyranosyl-(1→4)-0-(phenylmethyl 2,3-di-0-methyl-β-D-glucopyranuronosyluronate)-(1→4)-0-3,6-di-0-acetyl-2-0-phenylmethyl-α-D-glucopyranosyl-(1→4)-0-(phenylmethyl 2-0-benzoyl-3-0-octyl-α-L-idopyranuronosyluronate)-(1→4)-0-2,3,6-tri-0-benzoyl-α-D-glucopyranoside (0.051 mmol) was dissolved in methanol and the catalyst (10% Pd/C) was added. The mixture was stirred under an atmosphere of hydrogen for 2 days at room temperature then, after filtration, the solvent was evaporated to dryness to give methyl 0-6-0-acetyl-3,4-di-0-methyl-α-D-glucopyranosyl-(1→4)-0-2,3-di-0-methyl-β-D-glucopyranuronate-(1→4)-0-3,6-di-0-acetyl-α-D- glucopyranosyl-(1→4)-0-2-0-benzoyl-3-0-octyl-α-L-idopyranunonate-(1→4)-0-2,3,6-tri-0-benzoyl-α-D-glucopyranoside.
b. methyl 0-6-0-acetyl-3,4-di-0-methyl-α-D-glucopyranosyl-(1→4)-0-2,3-di-0-methyl-β-D-glucopyranuronate-(1→4)-0-3,6-di-0-acetyl-α-D-glucopyranosyl-(1→4)-0-2-0-benzoyl- 3-0-octyl-α-L-idopyranuronate)-(1→4)-0-2,3,6-tri-0-benzoyl-α-D-glucopyranoside was dissolved in methanol (8 ml) and sodium hydroxyde (5N solution) was added (0.9 ml). After 15 minutes at room temperature the solution was neutralized with Dowex-50-H⁺ resin, filtered and evaporated to dryness to give methyl 0-3,4-di-0-methyl-α-D-glucopyranosyl-(1→4)-0-2,3-di-0-methyl-β-D-glucopyranuronate-(1→4)-0-α-D-glucopyranosyl-(1→4)- 0-3-0-octyl-α-L-idopyranuronate-(1→4)- 0-α-D-glucopyranoside (48 mg).
c. methyl 0-3,4-di-0-methyl-α-D-glucopyranosyl-(1→4)-0-2,3-di-0-methyl-β-D-glucopyranuronate-(1→4)-0-α-D-glucopyranosyl-(1→4)- 0-3-0-octyl-α-L-idopyranuronate-(1→4)- 0-α-D-glucopyranoside (48 mg) was dissolved in dimethylformamide (1.4 ml) and sulfur trioxidetriethylamine complex (1.8 mmol) was added. After one night at 55 °C the mixture was cooled to room temperature and a mixture of sodium hydrogen carbonate (500 mg) and water (2 ml) was added. After evaporation the residue was layered on top of a Sephadex G25 column and eluted with water. The appropriate fractions were combined and freeze dried to give methyl 0-3,4-di-0-methyl-2,6-di-0-sulfo-α-D-glucopyranosyl-(1→4)-0-2,3-di-0-methyl-β-D-glucopyranuronosyl-(1→4)-0-2,3,6-tri-0-sulfo-α-D-glucopyranosyl-(1→4)- 0-3-0-octyl-2-0-sulfo-α-L-isopyranuronosyl-(1→4)-0-2,3,6-tri-0-sulfo-α-D-glucopyranoside undecakis sodium salt. [α]_{D}²⁰ = +40° (c 1.2; water). Anomeric protons chemical shifts: 4.68; 5.12; 5.13; 5.52; and 5.53 ppm.

### Example 4

In a similar manner, as described in Example 3, were prepared:
methyl 0-3,4-di-0-methyl-2,6-di-0-sulfo-α-D-glucopyranosyl-(1→4)-0-2,3-di-0-methyl-β-D-gluco-pyranuronosyl-(1→4)-0-2,3,6-tri-0-sulfo-α-D-gluco-pyranosyl-(1→4)- 0-3-0-butyl-2-0-sulfo-α-L-idopyranuronosyl-(1→4)-0-2,3,6-0-sulfo-α-D-gluco-pyranoside undecakis sodium salt.
[α]_{D}²⁰ = +40° (c 0.86; water).
methyl 0-3,4-di-0-methyl-2,6-di-0-sulfo-α-D-glucopyranosyl-(1→4)-0-2,3-di-0-butyl-β-D-glucopyranuronosyl-(1→4)-0-2,3,6-tri-0-sulfo-α-D-glucopyranosyl-(1-→4)-0-3- 0-octyl-2-0-sulfo-α-L-idopyranuronosyl-(1→4)-0-2,3,6-tri-0-sulfo-α-D-glucopyranoside undecakis sodium salt.
[α]_{D}²⁰ = +38° (c 0.95; water).
methyl 0-3,4-di-0-methyl-2,6-di-0-sulfo-α-D-glucopyranosyl-(1→4)-0-2,3-di-0-butyl-β-D-glucopyranuronosyl-(1→4)-0-2,3,6-tri-0-sulfo-α-D-glucopyranosyl-(1→4)-0-3-0- methyl-2-0-sulfo-α-L-idopyranuronosyl-(1→4)-0,2,3,6-tri-0-sulfo-α-D-glucopyranoside undecakis sodium salt.
[α]_{D}²⁰ = +43° (c 0.92; water).

## Claims (Claims for the following Contracting State(s): AT, BE, DE, DK, FR, GB, IT, NL, SE)

1. A carbohydrate derivative comprising a trisaccharide unit of the general formula I 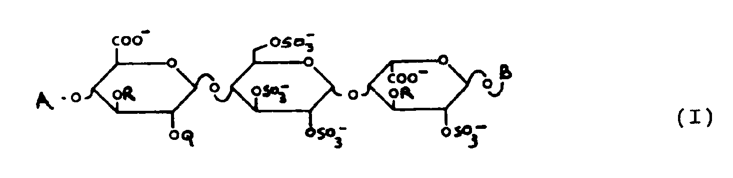 in which R is alkyl, aryl, or aralkyl;
Q is alkyl, aryl, aralkyl, or SO₃⁻;
A and B are independently selected from hydrogen, alkyl, aryl, aralkyl, or a carbohydrate group;
and the charged moieties are compensated by counter-ions.

2. The carbohydrate derivative according to claim 1, characterized in that R represents methyl and Q represents methyl.

3. The carbohydrate derivative according to claim 1, characterized in that R represents methyl and Q represents SO₃⁻

4. The carbohydrate derivative according to claim 2 or 3, characterized in that A represents: 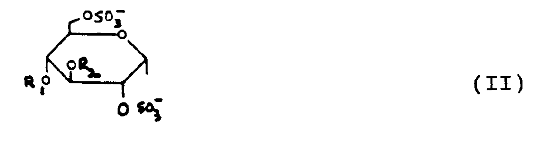 and B represents: 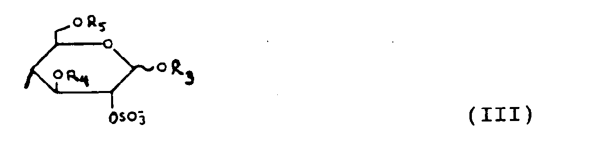 in which R₁, R₂, R₃, R₄ and R₅ are independently selected from hydrogen, alkyl, aryl, aralkyl, or SO₃⁻.

5. The carbohydrate derivative according to claim 4, characterized in that R₁, R₂, and R₃ are methyl, R₄ and R₅ are SO₃⁻, and OR₃ has the α-configuration.

6. A process for the preparation of the carbohydrate derivative of claim 1, characterized in that a protected carbohydrate derivative comprising a trisaccharide unit of the general formula: 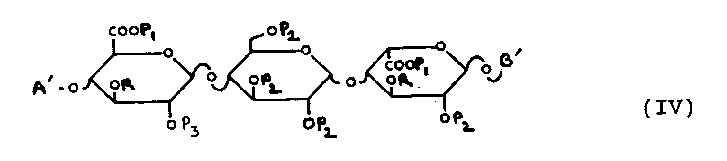 in which R is alkyl, aryl, or aralkyl, P₁ is an acid protective group, P₂ is a hydroxy protective group, P₃ is a hydroxy protective group or an alkyl, aryl, or aralkyl group, and A' and B' are A and B respectively, or protected derivatives thereof, is deprotected and sulfated in a manner known in the art.

7. A pharmaceutical composition comprising the carbohydrate derivative of claim 1, admixed with pharmaceutically acceptable auxiliaries.

8. A use of the carbohydrate derivative of claim 1 for the manufacture of a medicament for the treatment of thrombosis and inhibition of muscle cell proliferation.

9. The carbohydrate derivative according to any one of claims 1-5 for use in therapy.

## Claims (Claims for the following Contracting State(s): ES, GR)

1. A process for the preparation of a carbohydrate derivative comprising a trisaccharide unit having the general formula I 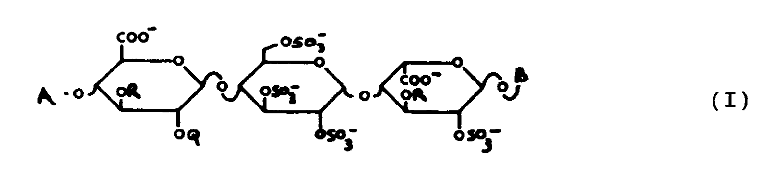 in which R is alkyl, aryl, or aralkyl;
Q is alkyl, aryl, aralkyl, or SO₃⁻;
A and B are independently selected from hydrogen, alkyl, aryl, aralkyl, or a carbohydrate group; and the charged moieties are compensated by counterions,
characterized in that a protected carbohydrate derivative comprising a trisaccharide unit of the general formula IV 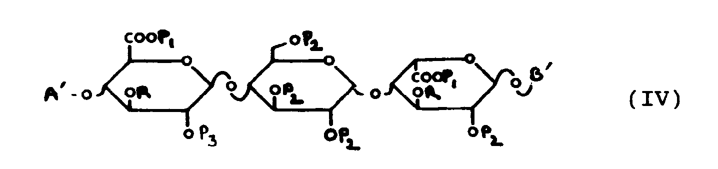 in which R is alkyl, aryl, or aralkyl, P₁ is an acid protective group, P₂ is a hydroxy protective group, P₃ is a hydroxy protective group or an alkyl, aryl, or aralkyl group, and A' and B' are A and B respectively, or protected derivatives thereof, is deprotected and sulfated in a manner known in the art.

2. The process according to claim 1, wherein R represents methyl and Q represents methyl.

3. The process according to claim 1, wherein R represents methyl and Q represents SO₃⁻.

4. The process according to claim 2 or 3, wherein A represents: 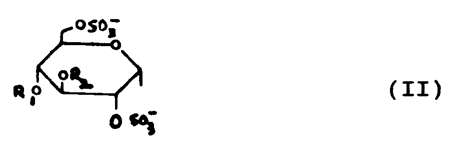 and B represents: 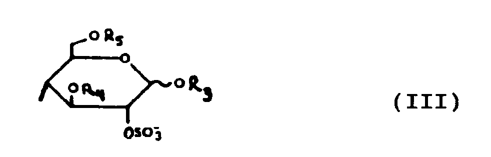 in which R₁, R₂, R₃, R₄ and R₅ are independently selected from hydrogen, alkyl, aryl, aralkyl, or SO₃⁻

5. The process according to claim 4, wherein R₁, R₂, and R₃ are methyl, R₄ and R₅ are SO₃⁻, and OR₃ has the α-configuration.

## Patentansprüche (Patentansprüche für folgende(n) Vertragsstaat(en): AT, BE, DE, DK, FR, GB, IT, NL, SE)

1. Ein Kohlehydratderivat, umfassend eine Trisaccharid-Einheit der allgemeinen Formel I 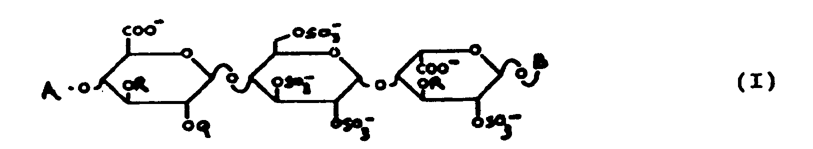 in welcher
R Alkyl, Aryl oder Aralkyl ist;
Q Alkyl, Aryl, Aralkyl oder SO₃⁻ ist;
A und B unabhängig voneinander ausgewählt sind aus Wasserstoff, Alkyl, Aryl, Aralkyl oder einer Kohlehydratgruppe;
und die geladenen Gruppierungen durch Gegenionen kompensiert sind.

2. Das Kohlehydratderivat nach Anspruch 1, dadurch gekennzeichnet, dass R Methyl und Q Methyl darstellen.

3. Das Kohlehydratderivat nach Anspruch 1, dadurch gekennzeichnet, dass R Methyl bedeutet und Q SO₃⁻ darstellt.

4. Das Kohlehydratderivat nach Anspruch 2 oder 3, dadurch gekennzeichnet, dass A 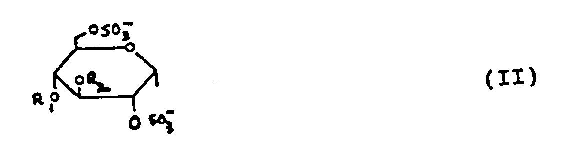 bedeutet und B 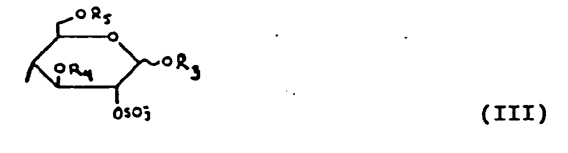 bedeutet, in welchen R₁ , R₂ , R₃ , R₄ und R₅ unabhängig voneinander ausgewählt sind aus Wasserstoff, Alkyl, Aryl, Aralkyl oder SO₃⁻.

5. Das Kohlehydratderivat nach Anspruch 4, dadurch gekennzeichnet, dass R₁ , R₂ und R₃ Methyl sind, R₄ und R₅ SO₃⁻ sind und OR₃ die α-Konfiguration aufweist.

6. Verfahren zur Herstellung des Kohlehydratderivates nach Anspruch 1, dadurch gekennzeichnet, dass ein geschütztes Kohlehydratderivat, welches eine Trisaccharideinheit der allgemeinen Formel IV 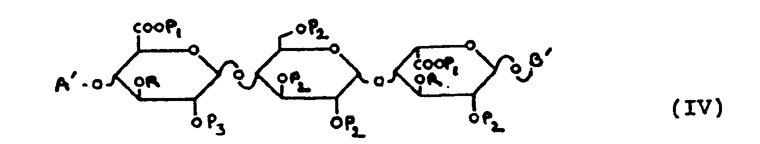 umfasst, in welcher R Alkyl, Aryl oder Aralkyl ist, P₁ eine Säureschutzgruppe ist, P₂ eine Hydroxyschutzgruppe ist, P₃ eine Hydroxyschutzgruppe oder eine Alkyl-, Aryl- oder Aralkylgruppe ist und A' und B' gleich A bzw. B sind oder geschützte Derivate davon, von der Schutzgruppe befreit und in einer in Fachkreisen bekannten Weise sulfatiert wird.

7. Pharmazeutische Zusammensetzung, umfassend das Kohlehydratderivat nach Anspruch 1, vermischt mit pharmazeutisch annehmbaren Hilfsstoffen.

8. Eine Verwendung des Kohlehydratderivates nach Anspruch 1 zur Herstellung eines Medikamentes für die Behandlung von Thrombose und für die Verhinderung von Muskelzelleproliferation.

9. Das Kohlehydratderivat nach einem der Ansprüche 1 bis 5 zur Verwendung in der Therapie.

## Patentansprüche (Patentansprüche für folgende(n) Vertragsstaat(en): GR, ES)

1. Verfahren zur Herstellung eines Kohlehydratderivates, welches eine Trisaccharideinheit der allgemeinen Formel I 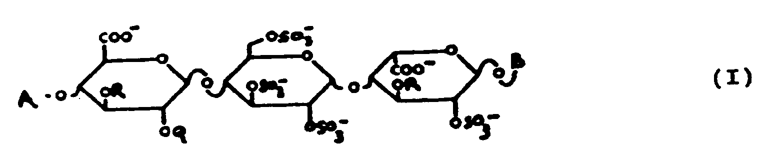 umfasst, in welcher
R Alkyl, Aryl oder Aralkyl ist;
Q Alkyl, Aryl, Aralkyl oder SO₃⁻ ist;
A und B unabhängig voneinander ausgewählt sind aus Wasserstoff, Alkyl, Aryl, Aralkyl oder einer Kohlehydratgruppe
und die geladenen Gruppierungen durch Gegenionen kompensiert sind, dadurch gekennzeichnet, dass ein geschütztes Kohlehydratderivat, welches eine Trisaccharideinheit der allgemeinen Formel IV 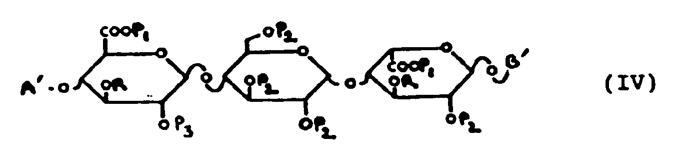 umfasst, in welcher
R Alkyl, Aryl oder Aralkyl ist,
P₁ eine Säureschutzgruppe ist,
P₂ eine Hydroxyschutzgruppe ist,
P₃ eine Hydroxyschutzgruppe oder eine Alkyl-, Aryl- oder Aralkylgruppe ist und
A' und B' gleich A bzw. B sind oder geschützte Derivate davon,
von der Schutzgruppe befreit und auf in Fachkreisen bekannte Weise sulfatiert wird.

2. Das Verfahren nach Anspruch 1, in welchem R Methyl bedeutet und Q Methyl bedeutet.

3. Verfahren nach Anspruch 1, in welchem R Methyl bedeutet und Q SO₃⁻ darstellt.

4. Das Verfahren nach Anspruch 2 oder 3, in welchem A 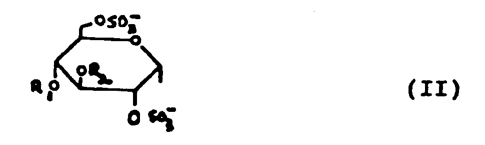 darstellt und B 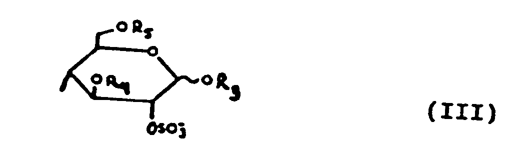 darstellt, in welchen R₁ , R₂ , R₃ , R₄ und R₅ unabhängig voneinander ausgewählt sind aus Wasserstoff, Alkyl, Aryl, Aralkyl oder SO₃⁻.

5. Das Verfahren nach Anspruch 4, in welchem R₁ , R₂ und R₃ Methyl sind, R₄ und R₅ SO₃⁻ sind und OR₃ die α-Konfiguration aufweisen.

## Revendications (Revendications pour l'(les) Etat(s) contractant(s) suivant(s): AT, BE, DE, DK, FR, GB, IT, NL, SE)

1. Un dérivé d'hydrate de carbone comprenant une unité trisaccharidique de formule générale I: 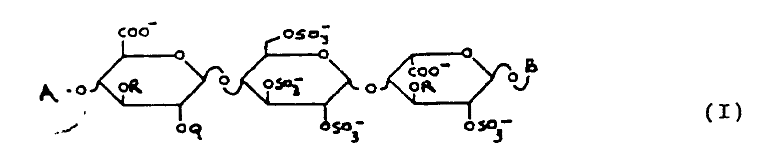 dans laquelle:
R est un radical alkyle, aryle ou aralkyle;
Q est un radical alkyle, aryle, aralkyle, ou SO₃⁻;
A et B sont choisis, indépendamment l'un de l'autre, dans le groupe comprenant l'atome d'hydrogène, les radicaux alkyle, aryle, aralkyle ou un résidu d'hydrate de carbone; les radicaux portant une charge étant compensée par des contre-ions.

2. Le dérivé d'hydrate de carbone selon la revendication 1, caractérisé en ce que R représente un radical méthyle et Q représente un radical méthyle.

3. Le dérivé d'hydrate de carbone selon la revendication 1, caractérisé en ce que R représente un radical méthyle et Q représente SO₃⁻.

4. Un dérivé d'hydrate de carbone selon les revendications 2 ou 3, caractérisé en ce que A représente: 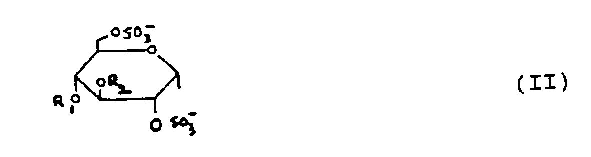 et B représente: 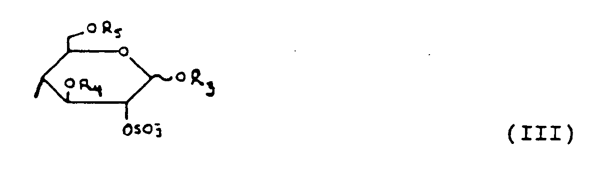 dans lesquelles:
R₁, R₂, R₃, R₄ et R₅ sont choisis, de façon indépendante, parmi l'hydrogène, les radicaux alkyle, aryle, aralkyle, ou SO₃⁻.

5. Le dérivé d'hydrate de carbone selon la revendication 4, caractérisé en ce que R₁, R₂ et R₃ sont des radicaux méthyles, R₄ et R₅ représentent SO₃⁻, et OR₃ présente une configuration α.

6. Un procédé pour la préparation d'un dérivé d'hydrate de carbone selon la revendication 1, caractérisé en ce que le dérivé d'hydrate de carbone protégé comprenant une unité trisaccharide de formule générale: 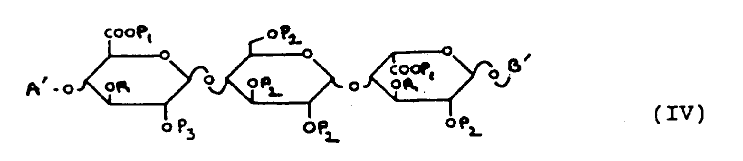 dans laquelle:
R est un radical alkyle, aryle ou aralkyle,
P₁ est un groupe protecteur acide,
P₂ est un groupe protecteur hydroxy,
P₃ est un groupe protecteur hydroxy ou un groupe alkyle, aryle ou aralkyle et
A' et B' sont respectivement A et B ou leurs dérivés protégés, est déprotégé et sulfaté selon une méthode connue de la technique

7. Une composition pharmaceutique comprenant des dérivés d'hydrate de carbone selon la revendication 1, mélangés à des auxiliaires pharmaceutiquement acceptables.

8. L'utilisation de dérivé d'hydrate de carbone selon la revendication 1, pour la fabrication d'un médicament pour le traitement de thrombose et l'inhibition de la prolifération des cellules musculaires.

9. Dérivé d'hydrate de carbone selon l'une quelconque des revendications 1 à 5, pour une utilisation en thérapeutique.

## Revendications (Revendications pour l'(les) Etat(s) contractant(s) suivant(s): ES, GR)

1. Un procédé pour la préparation d'un dérivé d'hydrate de carbone comprenant une unité trisaccharidique ayant la formule générale: 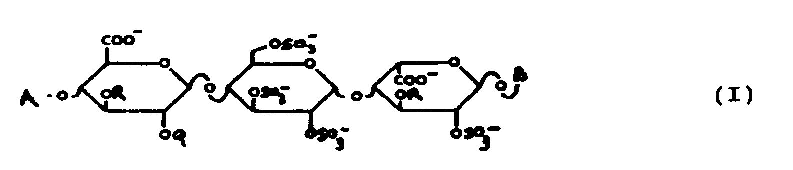 dans laquelle:
R est un radical alkyle, aryle ou aralkyle;
Q est un radical alkyle, aryle, aralkyle, ou SO₃⁻;
A et B sont choisis, indépendamment l'un de l'autre, dans le groupe contenant l'atome d'hydrogène, les radicaux alkyle, aryle, aralkyle ou un résidu d'hydrate de carbone; les radicaux portant une charge étant componsée par des contre-ions, caractérisé en ce que le dérivé d'hydrate de carbone protégé comprenant une unité trisaccharidique de formule générale IV 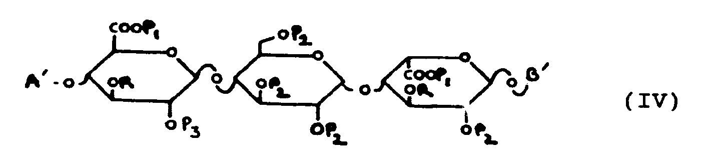
dans laquelle:
R est un radical alkyle, aryle ou aralkyle,
P₁ est un groupe protecteur acide,
P₂ est un groupe protecteur hydroxy,
P₃ est un groupe protecteur hydroxy ou un groupe alkyle, aryle ou aralkyle, et
A' et B' sont respectivement A et B ou leurs dérivés protégés,
est déprotégé et sulfaté selon une méthode connue de la technique.

2. Un procédé selon la revendication 1, dans lequel:
R représente un radical méthyle et
Q représente un radical méthyle.

3. Un procédé selon la revendication 1, dans lequel:
R représente un radical méthyle et
Q représente SO₃⁻.

4. Un procédé selon la revendication 2 ou 3, dans lequel:
A représente 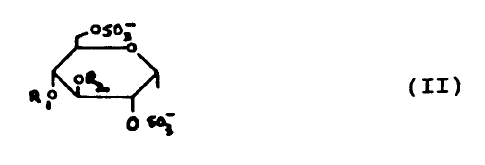 et B représente 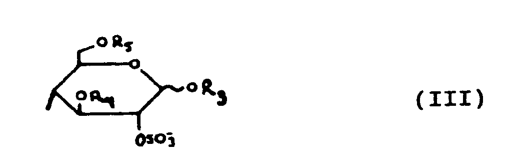 dans lesquels:
R₁, R₂, R₃, R₄ et R₅ sont choisis, indépendamment l'un de l'autre, parmi l'hydrogène, les groupes alkyle, aryle, aralkyle, ou SO₃⁻.

5. Un procédé selon la revendication 4 dans lequel R₁, R₂ et R₃ sont des radicaux méthyles, R₄ et R₅ sont SO₃⁻, et OR₃ a une configuration α.
